# EUROPEAN PATENT SPECIFICATION

(11) **EP 2 765 871 B1**
(45) Date of publication and mention of the grant of the patent: **26.07.2017**
(21) Application number: 12753327.1
(22) Date of filing: 20.08.2012
(51) Int. Cl.: A23L 33/00, A23L 33/10

(54) **COMPOSITION AND METHOD OF PHYTONUTRIENTS FOR METABOLIC PROGRAMMING EFFECTS**
ZUSAMMENSETZUNG UND VERFAHREN MIT PHYTONÄHRSTOFFEN ZUR PROGRAMMIERUNG VON STOFFWECHSELWIRKUNGEN
EFFETS PROGRAMMÉS GÉNÉTIQUES MÉTABOLIQUES D'UNE COMPOSITION CONTENANT DES PHYTONUTRIMENTS ET MÉTHODE ASSOCIÉE

(30) Priority: 14.10.2011 US 201113273652
(43) Date of publication of application: 20.08.2014
(73) Proprietor: MJN U.S. Holdings LLC, Glenview, IL 60026 (US)
(72) Inventor: JOUNI, Zeina,, Evansville, IN 47725 (US); HANLEY, Bryan,, Chicago, IL 60657 (US); CASSIDY, Aedin,, Norwich Norfolk NR4 7QF (GB)
(74) Representative: V.O.
(86) International application number: PCT/US2012/051519
(87) International publication number: WO 2013/055444

(56) References cited:
- WO-A1-2009/055585
- WO-A1-2009/105626
- US-A- 5 972 985
- US-A1- 2002 025 350
- US-A1- 2008 260 924
- US-B2- 6 989 161
- Dawn Marie: "EFFECTS OF THE PHYTOCHEMICALS QUERCETIN AND GENISTEIN ON THEPHASE I AND PHASE II ENZYME ACTIVITIES", Master of Science Thesis , 2003, pages 1-2, XP002689328, Retrieved from the Internet: URL:http://www.fcs.uga.edu/ss/docs/penn_da wn_m_200308_ms.pdf [retrieved on 2012-12-14]
- YOUNG-JOON SURH: "CANCER CHEMOPREVENTION WITH DIETARY PHYTOCHEMICALS", WWW.NATURE.COM/REVIEWS/CANCER, [Online] vol. 3, October 2003 (2003-10), pages 768-780, XP002689329, DOI: 10.1038/nrc1189 Retrieved from the Internet: URL:http://ww.w.drhuber.at/downloads/cance r_chemoprevention.pdf> [retrieved on 2012-12-14]
- ROBERT PREMIER: "Phytochemical composition: A paradigm shift forfood-health considerations", ASIA PACIFIC J CLIN NUTR, [Online] vol. 11, 2002, pages 197-201, XP002689330, Retrieved from the Internet: URL:http://apjcn.nhri.org.tw/server/apjcn/ Volume11/vol11sup2/S197.pdf> [retrieved on 2012-12-14]

## Description

### TECHNICAL FIELD

This disclosure relates to non-therapeutic methods of promoting phase II enzyme gene expression in pediatric subjects, the method comprising administering to a subject a composition comprising an effective amount of a phytonutrient. The present disclosure also relates to milk-based nutritional compositions for use in promoting phase II enzyme gene expression in pediatric subjects. More specifically, the present disclosure relates to nutritional compositions for pediatric subjects, especially infant formulas and growing-up milks, said products comprising phytonutrients, such as polyphenols, isothiocyanates, carotenoids, and mixtures thereof. The compositions are capable of promoting phase II enzyme gene expression in the subjects. The disclosure also relates to nutritional supplements for pregnant or lactating women, the supplements comprising the aforementioned phytonutrients. The supplements are capable of promoting phase II enzyme gene expression in prenatal infants of the pregnant women or in the infants nursing from the lactating women.

### BACKGROUND ART

Phytonutrients, such as polyphenols, carotenoids and isothiocyanates, are plant-derived bioactive compounds that have been associated with various health benefits in adults, including antioxidant activity, improved cardiovascular health, anti-inflammation, anti-aging, and neurological benefits. For example, phytonutrients are believed to provide important health benefits to humans, including protection against oxidative stress, inflammation, and many chronic and degenerative diseases.

For example, polyphenols, such as flavonoids, flavonols, flavones, isoflavones, anthocyanins and proanthocyanidins, have demonstrated antioxidant and anti-inflammatory activities consistent with promotion of vascular health, bone health and cognitive function. Similarly, carotenoids are known for their health benefits, particularly benefits to eye health. Certain carotenoids, such as lutein, zeaxanthin and lycopene are also being investigated for additional health benefits, including antioxidant activity, cardiovascular protection and eye and skin health. Moreover, isothiocyanates, which are found in cruciferous vegetables, are known for anticancer, antidiabetic and antimicrobial activity.

Furthermore, phytonutrients are present in human milk to varying degrees. For example, the carotenoid content of human milk has been the subject of many studies with the general conclusion that human milk content remains proportional to the mother's diet and correlates well to plasma carotenoid levels. Thus, breast-fed infants are routinely exposed to phytonutrients.

Although the mechanisms underlying the potential health benefits remain unclear, phytochemicals are thought to possess anti-inflammatory effects, act as cell signaling molecules, arrest cell cycle, and manipulate detoxification phase I and phase II enzymes. Phase I enzymes include cytochrome enzymes responsible for mixed function oxidase activity, while phase II enzymes are frequently involved in conjugation reactions necessary for drug metabolism or further metabolism of phase I enzyme products. At least 10 families of Phase I enzymes have been described in humans. Phase II metabolizing enzymes such as glutathione transferases (GSTs), UDP-glucuronosyltransferases (UGTs), sulfotransferases, N- & O- methyl transferases, and NAD(P)H:quinone oxidoreductase 1 (NQO1) enable the metabolism and eventual excretion of potentially harmful substances (xenobiotics) in adults. Phase II conjugation reactions generally follow Phase I activation, transforming harmful substances into water-soluble compounds that can be excreted through urine or bile. Several types of conjugation reactions are present in the body, including glucouronidation and sulfation. Prior to the present disclosure, there was no suggestion that dietary phytochemicals may modulate the expression of these phase II enzymes at different developmental stages in pediatric subjects, such as infants and children, to provide metabolic programming effects.
Penn DA (Effects of the phytochemicals quercetin and genistein on the phase I and phase II enzyme activities"; Master of Science Thesis) is directed to the investigation of the potential role of phytonutrients with respect to cancel and describes that and genistein increase phase II enzyme activity. Surh YJ (Nat Rev Cancer. 2003:3(10):768-80) is directed to cancer chemoprotective effects of dietary chemopreventatives and describes that a number phytochemicals has been shown to induce expression of phase II enzymes via NFR2.
Premier R (Asia Pacific J Clin Nutr. 2002;11(S6):S197-201) describes methods for increasing phytontrient concentrations in certain plant food products. It is described that isothiocyanates present in crucifer vegatables as glucosinilate precursors have been shown to have phase II enzyme inducer activity.

Metabolic programming (imprinting) has gained widespread acceptance over the last two decades, but many of the studies have concentrated on primary metabolic events leading to later stage obesity, and other metabolic disorders. The potential effect of early exposure to dietary components of phytochemicals has been comparatively little studied.

Despite the health benefits of fruit and vegetable consumption, dietary levels of phytonutrients in adults and children are often sub-optimal. Infants are not challenged with the majority of plant bioactive compounds until they are weaned, when non-milk based foods are introduced. But, the infant body needs to be able to express phase II metabolizing enzymes to prevent accumulation of potential toxins. Accordingly, there is a need to optimize nutrition for pediatric subjects, such as infants and or/children, at an early stage of development via the inclusion of phytonutrients in the diet of infants, children and/or pregnant and lactating women to achieve protective effects against harmful substances.

### DISCLOSURE OF THE INVENTION

In an embodiment, the present disclosure is directed to a non-therapeutic method of promoting phase II enzyme gene expression in a pediatric subject, comprising administering to the subject a nutritional composition having a composition as defined in any one of claims 1-11. In another embodiment, the present disclosure is directed to a non-therapeutic method of promoting phase II enzyme gene expression in an infant nursing from a lactating female, comprising administering to the lactating female a nutritional composition having a composition as defined in any one of claims 1-11, and feeding the infant with breast milk from the lactating female. In another embodiment, the present disclosure is directed to a method of promoting phase II enzyme gene expression in a prenatal infant, comprising administering to a female pregnant with the prenatal infant an effective amount of a nutritional composition having a composition as defined in any one of claims 1-11. In an embodiment, the aforementioned methods further promote and/or modulate phase II enzyme protein expression in the subject.

In one embodiment, the present disclosure is directed to a milk-based nutritional composition for use in promoting phase II enzyme gene expression in a pediatric subject, comprising a fat source, a carbohydrate source, a protein source and a phytonutrient source comprising a polyphenol, an isothiocyanate, a carotenoid or a mixture thereof, wherein the composition comprises 50 to 1300 nmol/L of the phytonutrient source. In another embodiment, the milk-based nutritional compositions are further capable of promoting phase II enzyme protein expression in a subject. The phytonutrient source may comprise a polyphenol, an isothiocyanate, a carotenoid or a mixture thereof. The nutritional composition may further comprise, among other ingredients, a source of long-chain polyunsaturated fatty acids, at least one prebiotic, a source of β-glucan, at least one probiotic, an amount of choline, a source of iron, or any combination thereof.

In another embodiment, the present disclosure is directed to a nutritional supplement for a pregnant or lactating female, the supplement comprising a nutritional composition having a composition as defined in any one of claims 1-11, wherein the supplement is capable of promoting phase II enzyme gene expression in a prenatal infant of the pregnant female or an infant nursing from the lactating female. In an embodiment, the supplement further promotes phase II enzyme protein expression. The phytonutrient source may comprise a polyphenol, an isothiocyanate, a carotenoid or a mixture thereof.

It is to be understood that both the foregoing general description and the following detailed description present embodiments of the disclosure and are intended to provide an overview or framework for understanding the nature and character of the disclosure as it is claimed. The description serves to explain the principles and operations of the claimed subject matter. Other and further features and advantages of the present disclosure will be readily apparent to those skilled in the art upon a reading of the following disclosure.

### BRIEF DESCRIPTION OF THE DRAWINGS

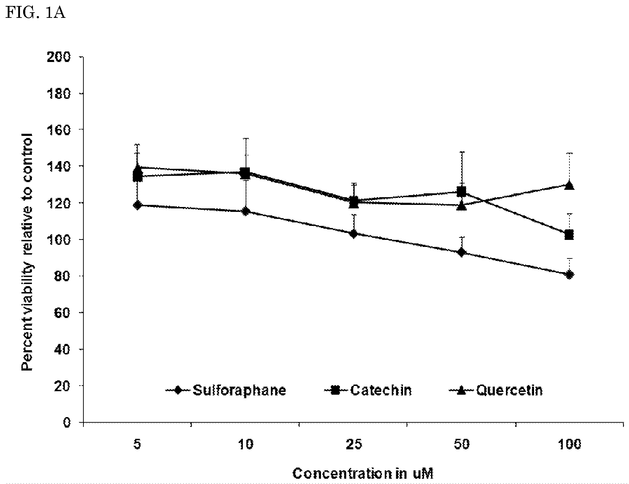

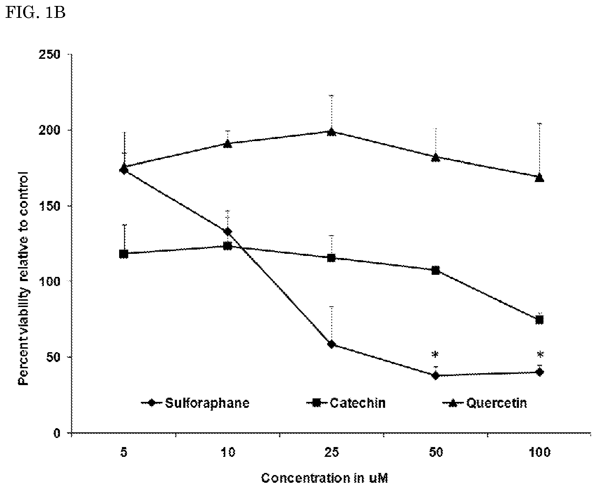

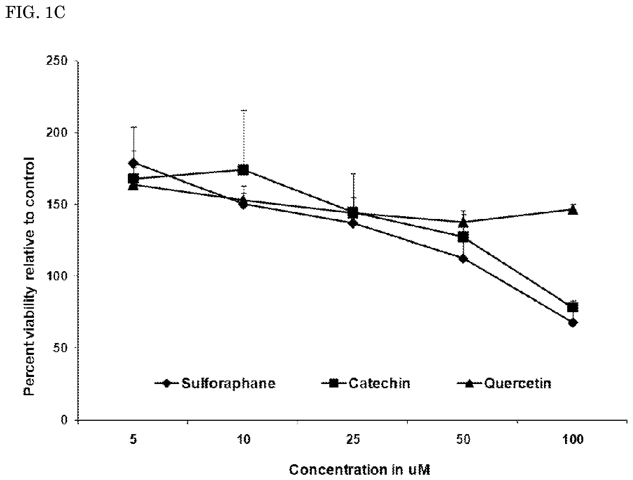

**FIGS. 1A****-C: Cell viability in 1 month old (A), 2 year old (B) and adult (C) cell models following phytochemical treatments.** FIGS. 1A-C depict cell viability in subjects of different ages following phytochemical treatment(s). More specifically, FIG. 1A depicts cell viability in a 1 month old cell model (FIG. 1A), FIG. 1B depicts cell viability in a 2 year old cell model (FIG. 1B), and FIG. 1C depicts cell viability in an adult cell model (FIG. 1C) in the presence of sulforaphane (◆), catechin (■) and quercetin (▲) compared to control cells. Following treatment with sulforaphane, significant loss of cell viability is observed in the 2 year old cell model (FIG. 1B) compared to control cells. No other significant changes in cell viability are observed in response to treatment with Catechin (■) or Quercetin (▲). Results are presented as percentage of control and represent mean ± SEM of at least 3 individual experiments. * indicates significantly decreased cell viability compared to control as assessed by one way ANOVA and post hoc t-test, p< 0.05.

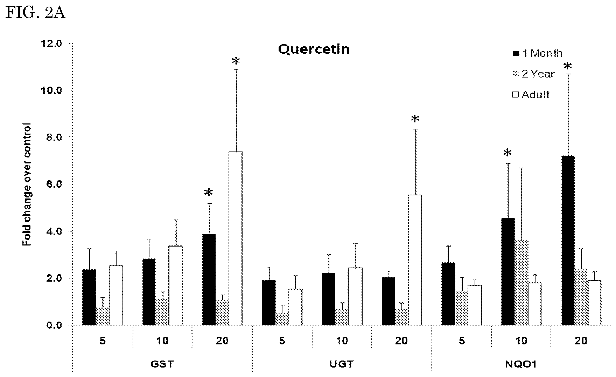

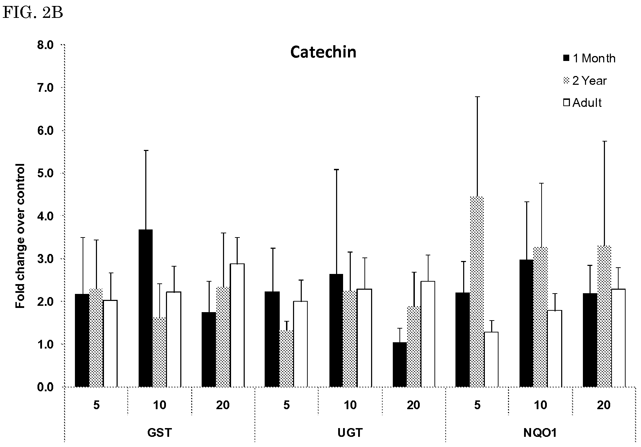

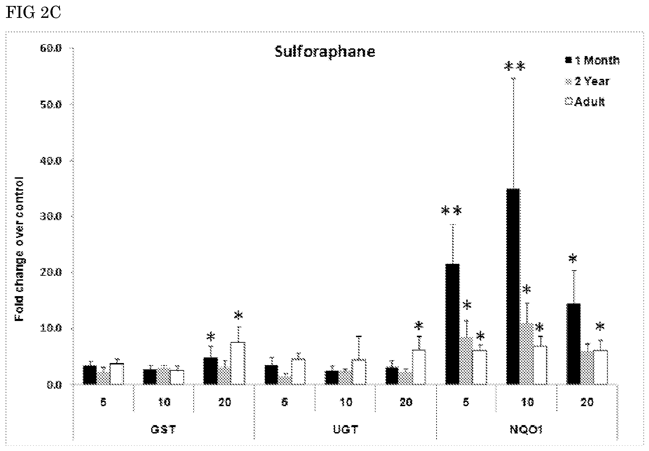

**FIGS. 2A****-C: Expression of mRNA in response to phytochemical treatment.** FIGS. 2A-C show the expression of GST, UGT and NQO1 mRNA in a one month (black bars), two year (gray bars) and adult (white bars) fibroblast cell model in the presence of varying concentrations of quercetin (FIG. 2A), catechin (FIG. 2B), and sulforaphane (FIG 2C). More specifically, FIG. 2A shows the expression of GST, UGT and NQO1 mRNA in a one month (black bar), two year (gray bar) and adult (white bar) fibroblast cell model in the presences of varying concentrations of quercetin. FIG. 2B shows the expression of GST, UGT and NQO1 mRNA in a one month (black bar), two year (gray bar) and adult (white bar) fibroblast cell model in the presences of varying concentrations of catechin. FIG. 2C shows the expression of GST, UGT and NQO1 mRNA in a one month (black bar), two year (gray bar) and adult (white bar) fibroblast cell model in the presences of varying concentrations of sulforaphane. The adult cell model demonstrates a significant dose-dependent increase in both GST and UGT mRNA expression upon treatment with quercetin. Expression of GST and NQO1 mRNA is significantly increased within the 1 month old cell model. Following catechin treatment, the infant cell models demonstrate significant increases in GST and NQO1 mRNA. In the adult cell line, a significant increase in GST, UGT and NQO1 mRNA is observed upon treatment with sulforaphane, in addition to significant increases in GST and NQO1 in the infant cell models. Results are mean ± SEM of at least 3 individual experiments, and normalized against control=1. * & ** indicates significantly increased expression relative to control as assessed by one way ANOVA and post hoc t-test, p< 0.05 and p<0.01 respectively.

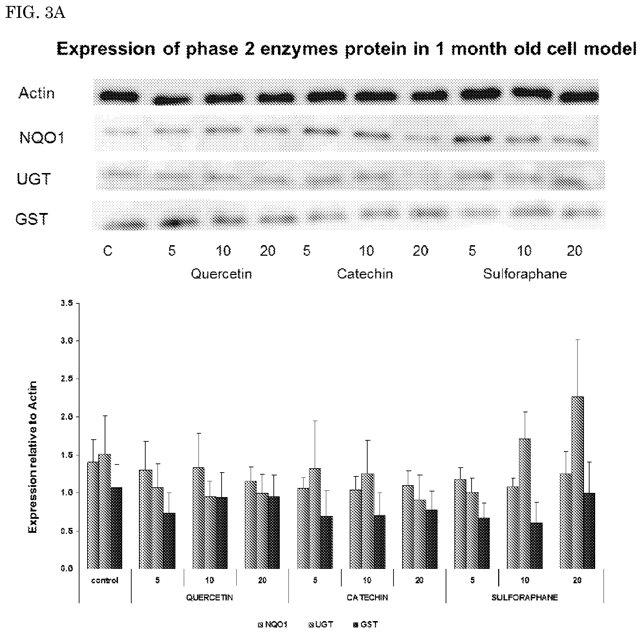

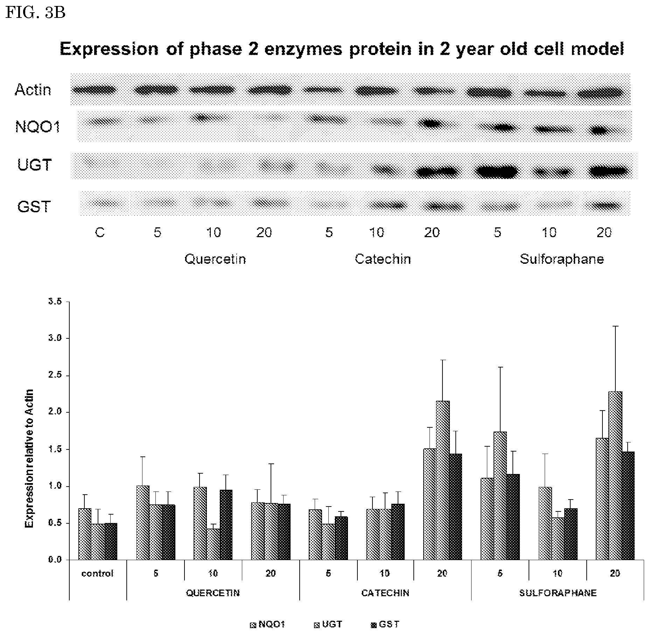

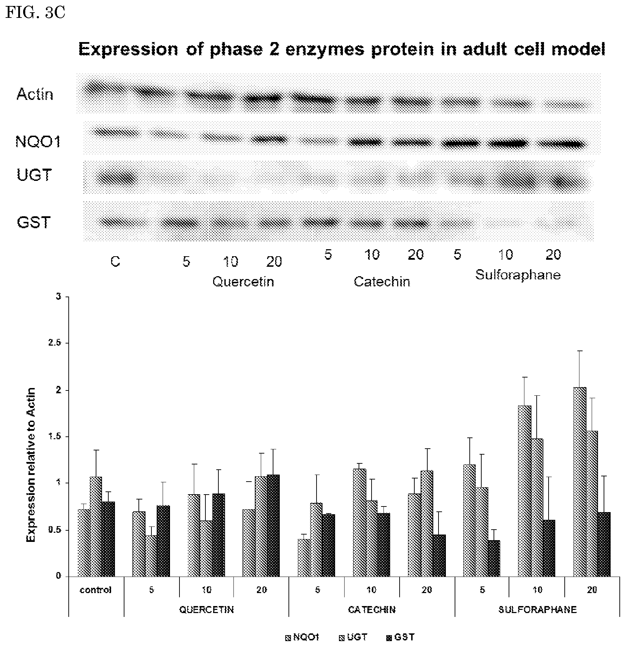

**FIG 3A****-C: Effect of phytochemical treatment on phase II enzyme protein expression.** FIG. 3A depicts immunoblotting for effect of 24h phytochemical treatment (control, 5, 10 and 20µM as indicated) and protein expression of phase II enzymes NQO1 (medium gray bars), UGT (light gray bars) and GST (black gray bars) in a 1 month old cell model. FIG. 3B depicts immunoblotting for effect of 24h phytochemical treatment and protein expression of phase II enzymes in a 2 year old cell model. FIG. 3C depicts immunoblotting for effect of 24h phytochemical and protein expression of phase II enzymes in an adult cell model. All membranes are stripped and re-probed for anti-β-actin antibody to ensure equal loading. Experiments are repeated at least 3 times and results are relative ratio to beta-actin.

### BEST MODE FOR CARRYING OUT THE INVENTION

Reference now will be made in detail to the embodiments of the present disclosure, one or more examples of which are set forth herein below. Each example is provided by way of explanation of the nutritional composition of the present disclosure and is not a limitation. In fact, it will be apparent to those skilled in the art that various modifications and variations can be made to the teachings of the present disclosure without departing from the scope of the disclosure. For instance, features illustrated or described as part of one embodiment, can be used with another embodiment to yield a still further embodiment.

Thus, it is intended that the present disclosure covers such modifications and variations as come within the scope of the appended claims and their equivalents. Other objects, features and aspects of the present disclosure are disclosed in or are obvious from the following detailed description. It is to be understood by one of ordinary skill in the art that the present discussion is a description of exemplary embodiments only and is not intended as limiting the broader aspects of the present disclosure.

"Nutritional composition" means a substance or formulation that satisfies at least a portion of a subject's nutrient requirements. The terms "nutritional(s)", "nutritional formula(s)", "enteral nutritional(s)", "nutritional composition(s)", and "nutritional supplement(s)" are used interchangeably throughout the present disclosure to refer to liquids, powders, gels, pastes, solids, concentrates, suspensions, or ready-to-use forms of enteral formulas, oral formulas, formulas for infants, formulas for pediatric subjects, formulas for children, growing-up milks and/or formulas for adults, such as women who are lactating or pregnant.

The term "enteral" means through or within the gastrointestinal, or digestive, tract. "Enteral administration" includes oral feeding, intragastric feeding, transpyloric administration, or any other administration into the digestive tract.

"Pediatric subject" means a human that is less than 13 years of age. In some embodiments, a pediatric subject refers to a human subject that is less than 8 years old. In other embodiments, a pediatric subject refers to a human subject between 1 and 6 years of age. In still further embodiments, a pediatric subject refers to a human subject between 6 and 12 years of age.

"Infant" means a subject having an age of not more than about one year and includes infants from 0 to about 12 months. The term infant includes low birth weight infants, very low birth weight infants, and preterm infants. "Preterm" means an infant born before the end of the 37^{th} week of gestation. The term infant also includes prenatal infants, e.g., infants still in *utero.*

"Child" means a subject ranging in age from about 12 months to about 13 years. In some embodiments, a child is a subject between the ages of 1 and 12 years old. In other embodiments, the terms "children" or "child" refer to subjects that are between about one and about six years old, or between about seven and about 12 years old. In other embodiments, the terms "children" or "child" refer to any range of ages between about 12 months and about 13 years.

"Children's nutritional product" refers to a composition that satisfies at least a portion of the nutrient requirements of a child. A growing-up milk is an example of a children's nutritional product.

"Infant formula" means a composition that satisfies at least a portion of the nutrient requirements of an infant. In the United States, the content of an infant formula is dictated by the federal regulations set forth at 21 C.F.R. Sections 100, 106, and 107. These regulations define macronutrient, vitamin, mineral, and other ingredient levels in an effort to simulate the nutritional and other properties of human breast milk.

The term "growing-up milk" refers to a broad category of nutritional compositions intended to be used as a part of a diverse diet in order to support the normal growth and development of a child between the ages of about 1 and about 6 years of age.

"Milk-based" means comprising at least one component that has been drawn or extracted from the mammary gland of a mammal. In some embodiments, a milk-based nutritional composition comprises components of milk that are derived from domesticated ungulates, ruminants or other mammals or any combination thereof. Moreover, in some embodiments, milk-based means comprising bovine casein, whey, lactose, or any combination thereof. Further, "milk-based nutritional composition" may refer to any composition comprising any milk-derived or milk-based product known in the art.

"Nutritionally complete" means a composition that may be used as the sole source of nutrition, which would supply essentially all of the required daily amounts of vitamins, minerals, and/or trace elements in combination with proteins, carbohydrates, and lipids. Indeed, "nutritionally complete" describes a nutritional composition that provides adequate amounts of carbohydrates, lipids, essential fatty acids, proteins, essential amino acids, conditionally essential amino acids, vitamins, minerals and energy required to support normal growth and development of a subject.

Therefore, a nutritional composition that is "nutritionally complete" for a preterm infant will, by definition, provide qualitatively and quantitatively adequate amounts of carbohydrates, lipids, essential fatty acids, proteins, essential amino acids, conditionally essential amino acids, vitamins, minerals, and energy required for growth of the preterm infant.

A nutritional composition that is "nutritionally complete" for a term infant will, by definition, provide qualitatively and quantitatively adequate amounts of all carbohydrates, lipids, essential fatty acids, proteins, essential amino acids, conditionally essential amino acids, vitamins, minerals, and energy required for growth of the term infant.

A nutritional composition that is "nutritionally complete" for a child will, by definition, provide qualitatively and quantitatively adequate amounts of all carbohydrates, lipids, essential fatty acids, proteins, essential amino acids, conditionally essential amino acids, vitamins, minerals, and energy required for growth of a child.

As applied to nutrients, the term "essential" refers to any nutrient that cannot be synthesized by the body in amounts sufficient for normal growth and to maintain health and that, therefore, must be supplied by the diet. The term "conditionally essential" as applied to nutrients means that the nutrient must be supplied by the diet under conditions when adequate amounts of the precursor compound is unavailable to the body for endogenous synthesis to occur.

"Nutritional supplement" or "supplement" refers to a formulation that contains a nutritionally relevant amount of at least one nutrient. For example, supplements described herein may provide at least one nutrient for a human subject, such as a lactating or pregnant female.

"Probiotic" means a microorganism with low or no pathogenicity that exerts a beneficial effect on the health of the host.

"Prebiotic" means a non-digestible food ingredient that beneficially affects the host by selectively stimulating the growth and/or activity of one or a limited number of beneficial gut bacteria in the digestive tract, selective reduction in gut pathogens, or favorable influence on gut short chain fatty acid profile that can improve the health of the host.

"Phytonutrient" means a chemical compound that occurs naturally in plants. The term "phytonutrient(s)" encompasses several broad categories of compounds produced by plants, such as, for example, polyphenolic compounds, such as flavonoids, flavonols, flavones, isoflavonones, flavan-3-ols, isoflavonoids, anthocyanins, proanthocyanins, catechins, and epicathicins. Phytonutrients also encompass carotenoids, phytosterols, thiols, isothiocyanates, and other plant-derived compounds.

"β-glucan" means all β-glucan, including both β-1,3-glucan and β-1,3;1,6-glucan, as each is a specific type of β-glucan. Moreover, β-1,3;1,6-glucan is a type of β-1,3-glucan. Therefore, the term "β-1,3-glucan" includes β-1,3;1,6-glucan.

All percentages, parts and ratios as used herein are by weight of the total formulation, unless otherwise specified.

The nutritional composition of the present disclosure may be free of substantially free of any optional or selected ingredients described herein. In this context, and unless otherwise specified, the term "substantially free" means that the selected composition may contain less than a functional amount of the optional ingredient, typically less than 0.1% by weight, and also, including zero percent by weight of such optional or selected ingredient.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The methods and compositions of the present disclosure, including components thereof, can comprise, consist of, or consist essentially of the essential elements and limitations of the embodiments described herein, as well as any additional or optional ingredients, components or limitations described herein or otherwise useful in nutritional compositions.

As used herein, the term "about" should be construed to refer to both of the numbers specified in any range. Any reference to a range should be considered as providing support for any subset within that range.

The present disclosure provides nutritional compositions comprising a phytonutrient source. The nutritional compositions further comprise a protein source, a carbohydrate source, and a fat or lipid source. More specifically, the present disclosure provides a milk-based nutritional composition, comprising a fat source, a carbohydrate source, a protein source and a phytonutrient source.

The nutritional composition of the present disclosure includes at least one phytonutrient. The phytonutrient source may be derived from a fruit or vegetable, or, in certain embodiments, the phytonutrient source may be chemically derived or synthetically made. The phytonutrient source comprises a polyphenol, a carotenoid, an isothiocyanate, or a mixture thereof.

For the purposes of this disclosure, phytonutrients may be added to a nutritional composition in their native, purified, encapsulated and/or chemically or enzymatically-modified form so as to deliver the desired sensory and stability properties. In the case of encapsulation, it is desirable that the encapsulated phytonutrients resist dissolution with water but are released upon reaching the small intestine. This could be achieved by the application of enteric coatings, such as cross-linked alginate and others. Furthermore, the nutritional composition may comprise the metabolite(s) of a phytonutrient or of its parent compound.

Polyphenols suitable for use in the nutritional compositions described herein include, without limitation, anthocyanins, anthocyanins, proanthocyanidins, cyanidins, flavanols, flavonols, flavan-3-ols, flavones, flavanones, and isoflavonoids. For example, the polyphenols include epicatechins, catechins, resveratrol, quercetin, curcumin, or any mixture thereof, as well as any possible combination of the phytonutrients in a purified or natural form.

In some embodiments, the anthocyanins, may be, without limitation, glucosides of aurantinidin, cyanidin, delphinidin, europinidin, luteolinidin, pelargonidin, malyidin, peonidin, petunidin, and rosinidin. These and other anthocyanins suitable for use in the nutritional composition are found in a variety of plant sources. Anthocyanins may be derived from a single plant source or a combination of plant sources. Non-limiting examples of plants rich in anthocyanins suitable for use in the inventive composition include: berries (acai, grape, bilberry, blueberry, lingonberry, black currant, chokeberry, blackberry, raspberry, cherry, red currant, cranberry, crowberry, cloudberry, whortleberry, rowanberry), purple corn, purple potato, purple carrot, red sweet potato, red cabbage, eggplant.

Proanthocyanidins suitable for use in the nutritional composition described herein include, without limitation, polymers of flavan-3-ols (e.g., catechins, epicatechins) with degrees of polymerization in the range of 2 to 11. Such compounds may be derived from a single plant source or a combination of plant sources. Non-limiting examples of plant sources rich in proanthocyanidins suitable for use in the inventive nutritional composition include: grape, grape skin, grape seed, green tea, black tea, apple, pine bark, cinnamon, cocoa, bilberry, cranberry, black currant chokeberry.

Non-limiting examples of flavanols that are suitable for use in the inventive nutritional composition include catechin, epicatechin, epigallocatechin, epicatechin gallate, epigallocatechin gallate, quercetin, myricetin, kaempferol or any mixture thereof. Plants rich in the suitable flavan-3-ols and flavonols include, but are not limited to, apple, grape seed, grape, grape skin, tea (green or black), pine bark, cinnamon, cocoa, bilberry, cranberry, black currant, chokeberry, orange, lime and lemon.

In some embodiments, the nutritional composition is formulated to deliver an amount of flavan-3-ols (such as catechins) that may range from about 0.1 to about 170 mg/day. In other embodiments, the nutritional composition is formulated to deliver an amount of flavan-3-ols that may range from about 0.01 and about 150 mg/day. And in certain embodiments, the nutritional composition comprises between about 0.01 and about 338 mg flavan-3-ols per liter. The amount of flavonols, such as quercetin, may range from about 50 to about 400 nmol/L. In certain embodiments, the nutritional composition comprises between about 0.01 and about 211 mg flavonols per liter. And in some embodiments the nutritional composition is formulated to deliver an amount of flavonols (such as quercetin) that may range from about 0.1 to about 150 mg/day. If the nutritional composition is directed to or formulated for administration to an infant from about 0 to about 12 months of age, the amount of catechins may range from about 500 to about 1300 nmol/L. In some embodiments, the nutritional composition may be formulated to deliver between about 0.01 and about 50 mg flavan-3-ols, such as catechins, per day. The amount of quercetin may range from about 50 to about 200 nmol/L. In some embodiments, the nutritional composition may be formulated to deliver between about 0.01 and about 40 mg flavonols, such as quercetin, per day.

In some embodiments, the nutritional composition of the present disclosure comprises flavanones and/or flavones. Non-limiting examples of suitable flavanones include butin, eriodictyol, hesperetin, hesperidin, homeriodictyol, isosakuranetin, naringenin, naringin, pinocembrin, poncirin, sakuranetin, sakuranin, steurbin. Non-limiting examples of suitable flavones include apigenin and luteolin. Plant sources rich in flavanones and/or flavones include, but are not limited to orange, tangerine, grapefruit, lemon, lime, celery, parsley, and capsicum pepper. Moreover, the nutritional composition may also comprise flavonoids. Flavonoids from plant or algae extracts may be useful in the monomer, dimer and/or polymer forms.

The nutritional composition may also comprise isoflavonoids. Examples of isoflavonoids include, but are not limited to, genistein (genistin), daidzein (daidzin), biochanin A, formononetin, coumestrol, irilone, orobol, pseudobaptigenin, anagyroidisoflavone A and B, calycosin, glycitein, irigenin, 5-O- methylgenistein, pratensein, prunetin, psi-tectorigenin, retusin, tectorigenin, iridin, ononin, puerarin, tectoridin, derrubone, luteone, wighteone, alpinumisoflavone, barbigerone, di-O-methylalpinumisoflavone, 4'-methylalpinumisoflavone. Plant sources rich in isoflavonoids, include, but are not limited to, psoralea, kudzu, lupine, fava, chick pea, alfalfa, and peanut. In certain embodiments, the nutritional composition may be free of or substantially free of soy isoflavonoids. In some embodiments, the nutritional composition is free of or substantially free of soy phytonutrients, such as soy isoflavonoids.

The nutritional composition may comprise other classes of dietary phytonutrients, such as glucosinolate or isothiocyanates. Representative isothiocyanates include, without limitation, sulforaphane and phenethylisothiocyanate. Plant sources rich in isothiocyanates include cruciferous vegetables, such as Brussels sprouts, cabbage, cauliflower, bok choy, kale, collards, Chinese broccoli, broccoli raab, kohlrabi, mustard, turnip, radish, arugula, watercress and mixtures thereof.

In certain embodiments, the nutritional composition comprises carotenoids, such as lutein, zeaxanthin, astaxanthin, lycopene, beta-carotene, alpha-carotene, gamma-carotene, and/or beta-cryptoxanthin. Plant sources rich in carotenoids include, but are not limited to kiwi, grapes, citrus, tomatoes, watermelons, papayas and other red fruits, or dark greens, such as kale, spinach, turnip greens, collard greens, romaine lettuce, broccoli, zucchini, garden peas and Brussels sprouts, spinach, carrots, and other red, orange or yellow fruits and vegetables.

In some embodiments, the nutritional composition is a fortified, milk-based nutritional composition, such as an infant formula or a growing-up milk, which comprises at least one phytonutrient. The nutritional composition may be capable of metabolic programming of phase II enzymes in pediatric subject. The nutritional composition is capable of promoting phase II enzyme gene expression in a pediatric subject. In some embodiments, the nutritional composition may be capable of modulating phase II enzyme protein expression in a pediatric subject.

The disclosed nutritional composition(s) may be provided in any form known in the art, such as a powder, a gel, a suspension, a paste, a solid, a liquid, a liquid concentrate, a reconstituteable powdered milk substitute or a ready-to-use product. The nutritional composition may, in certain embodiments, comprise a nutritional supplement, children's nutritional product, infant formula, human milk fortifier, growing-up milk or any other nutritional composition designed for a pediatric subject. Nutritional compositions of the present disclosure include, for example, orally-ingestible, health-promoting substances including, for example, foods, beverages, tablets, capsules and powders. Moreover, the nutritional composition of the present disclosure may be standardized to a specific caloric content, it may be provided as a ready-to-use product, or it may be provided in a concentrated form. In some embodiments, the nutritional composition is in powder form with a particle size in the range of 5 µm to 1500 µm, more preferably in the range of 10 µm to 1000µm, and even more preferably in the range of 50 µm to 300µm.

In some embodiments, the disclosure provides a fortified milk-based growing-up milk designed for children ages 1-3 years and/or 4-6 years, wherein the growing-up milk supports growth and development and life-long health. In some embodiments, the disclosure provides an infant formula suitable for infants ranging in age from 0 to 12 months, or from 0 to 3 months, 0 to 6 months or 6 to 12 months.

Suitable fat or lipid sources for the nutritional composition of the present disclosure may be any known or used in the art, including but not limited to, animal sources, e.g., milk fat, butter, butter fat, egg yolk lipid; marine sources, such as fish oils, marine oils, single cell oils; vegetable and plant oils, such as corn oil, canola oil, sunflower oil, soybean oil, palm olein oil, coconut oil, high oleic sunflower oil, evening primrose oil, rapeseed oil, olive oil, flaxseed (linseed) oil, cottonseed oil, high oleic safflower oil, palm stearin, palm kernel oil, wheat germ oil; medium chain triglyceride oils and emulsions and esters of fatty acids; and any combinations thereof.

Carbohydrate sources can be any used in the art, e.g., lactose, glucose, fructose, corn syrup solids, maltodextrins, sucrose, starch, rice syrup solids, and the like. The amount of carbohydrate in the nutritional composition typically can vary from between about 5 g and about 25 g/100 kcal.

The nutritional composition(s) of the disclosure also comprise a protein source. The protein source can be any used in the art, e.g., nonfat milk, whey protein, casein, soy protein, hydrolyzed protein, amino acids, and the like. Bovine milk protein sources useful in practicing the present disclosure include, but are not limited to, milk protein powders, milk protein concentrates, milk protein isolates, nonfat milk solids, nonfat milk, nonfat dry milk, whey protein, whey protein isolates, whey protein concentrates, sweet whey, acid whey, casein, acid casein, caseinate (*e.g*. sodium caseinate, sodium calcium caseinate, calcium caseinate) and any combinations thereof.

In one embodiment, the proteins of the nutritional composition are provided as intact proteins. In other embodiments, the proteins are provided as a combination of both intact proteins and partially hydrolyzed proteins. In certain other embodiments, the proteins are more completely hydrolyzed. In still other embodiments, the protein source comprises amino acids. In yet another embodiment, the protein source may be supplemented with glutamine-containing peptides.

In a particular embodiment of the nutritional composition, the whey:casein ratio of the protein source is similar to that found in human breast milk. In an embodiment, the protein source comprises from about 40% to about 80% whey protein and from about 20% to about 60% casein.

In some embodiments, the nutritional composition comprises between about 1 g and about 7 g of a protein source per 100 kcal.

In one embodiment, the nutritional composition may contain one or more probiotics. Any probiotic known in the art may be acceptable in this embodiment. In a particular embodiment, the probiotic may be selected from any *Lactobacillus* species, *Lactobacillus rhamnosus* GG (ATCC number 53103), *Bifidobacterium* species, *Bifidobacterium longum,* and *Bifidobacterium animalis subsp. lactis* BB-12 (DSM No. 10140) or any combination thereof.

If included in the composition, the amount of the probiotic may vary from about 1 x 10⁴ to about 1 x 10¹⁰ colony forming units (cfu) per kg body weight per day. In another embodiment, the amount of the probiotic may vary from about 10⁶ to about 10¹⁰ cfu per kg body weight per day. In still another embodiment, the amount of the probiotic may vary from about 10⁷ to about 10⁹ cfu per day. In yet another embodiment, the amount of the probiotic may be at least about 10⁶ cfu per day.

In an embodiment, the probiotic(s) may be viable or non-viable. As used herein, the term "viable", refers to live microorganisms. The term "non-viable" or "non-viable probiotic" means non-living probiotic microorganisms, their cellular components and/or metabolites thereof. Such non-viable probiotics may have been heat-killed or otherwise inactivated, but they retain the ability to favorably influence the health of the host. The probiotics useful in the present disclosure may be naturally-occurring, synthetic or developed through the genetic manipulation of organisms, whether such new source is now known or later developed.

The nutritional composition may also contain one or more prebiotics in certain embodiments. Such prebiotics may be naturally-occurring, synthetic, or developed through the genetic manipulation of organisms and/or plants, whether such new source is now known or developed later. Prebiotics useful in the present disclosure may include oligosaccharides, polysaccharides, and other prebiotics that contain fructose, xylose, soya, galactose, glucose and mannose.

More specifically, prebiotics useful in the present disclosure may include polydextrose, polydextrose powder, lactulose, lactosucrose, raffinose, gluco-oligosaccharide, inulin, fructo-oligosaccharide, isomalto-oligosaccharide, soybean oligosaccharides, lactosucrose, xylo-oligosaccharide, chito-oligosaccharide, manno-oligosaccharide, aribino-oligosaccharide, siallyl-oligosaccharide, fuco-oligosaccharide, galacto-oligosaccharide, and gentio-oligosaccharides.

In an embodiment, the total amount of prebiotics present in the nutritional composition may be from about 1.0 g/L to about 10.0 g/L of the composition. At least 20% of the prebiotics can comprise galacto-oligosaccharide, polydextrose or a mixture thereof. The amount of each of galacto-oligosaccharide and/or polydextrose in the nutritional composition may, in an embodiment, be within the range of from about 1.0 g/L to about 4.0 g/L.

The nutritional composition of the disclosure may contain a source of long chain polyunsaturated fatty acid (LCPUFA) that comprises docosahexaenoic acid. Other suitable LCPUFAs include, but are not limited to, α-linoleic acid, γ-linoleic acid, linoleic acid, linolenic acid, eicosapentaenoic acid (EPA) and arachidonic acid (ARA).

In an embodiment, especially if the nutritional composition is an infant formula, the nutritional composition is supplemented with both DHA and ARA. In this embodiment, the weight ratio of ARA:DHA may be between about 1:3 and about 9:1. In a particular embodiment, the ratio of ARA:DHA is from about 1:2 to about 4:1.

The nutritional composition may be supplemented with oils containing DHA and/or ARA using standard techniques known in the art. For example, DHA and ARA may be added to the composition by replacing an equivalent amount of an oil, such as high oleic sunflower oil, normally present in the composition. As another example, the oils containing DHA and ARA may be added to the composition by replacing an equivalent amount of the rest of the overall fat blend normally present in the composition without DHA and ARA.

If included, the source of DHA and/or ARA may be any source known in the art such as marine oil, fish oil, single cell oil, egg yolk lipid, and brain lipid. In some embodiments, the DHA and ARA are sourced from single cell Martek oils, DHASCO® and ARASCO®, or variations thereof. The DHA and ARA can be in natural form, provided that the remainder of the LCPUFA source does not result in any substantial deleterious effect on the subject. Alternatively, the DHA and ARA can be used in refined form.

In an embodiment, sources of DHA and ARA are single cell oils as taught in U.S. Pat. Nos. 5,374,657; 5,550,156; and 5,397,591. Nevertheless, the present disclosure is not limited to only such oils.

The nutritional composition may also comprise a source of β-glucan. Glucans are polysaccharides, specifically polymers of glucose, which are naturally occurring and may be found in cell walls of bacteria, yeast, fungi, and plants. Beta glucans (β-glucans) are themselves a diverse subset of glucose polymers, which are made up of chains of glucose monomers linked together via beta-type glycosidic bonds to form complex carbohydrates.

β-1,3-glucans are carbohydrate polymers purified from, for example, yeast, mushroom, bacteria, algae, or cereals. (Stone BA, Clarke AE. Chemistry and Biology of (1-3)-Beta-Glucans. London:Portland Press Ltd; 1993.) The chemical structure of β-1,3-glucan depends on the source of the β-1,3-glucan. Moreover, various physiochemical parameters, such as solubility, primary structure, molecular weight, and branching, play a role in biological activities of β-1,3-glucans. (Yadomae T., Structure and biological activities of fungal beta-1,3-glucans. Yakugaku Zasshi. 2000;120:413-431.)

β-1,3-glucans are naturally occurring polysaccharides, with or without β-1,6-glucose side chains that are found in the cell walls of a variety of plants, yeasts, fungi and bacteria. B-1,3;1,6-glucans are those containing glucose units with (1,3) links having side chains attached at the (1,6) position(s). β-1,3;1,6 glucans are a heterogeneous group of glucose polymers that share structural commonalities, including a backbone of straight chain glucose units linked by a β-1,3 bond with β-1,6-linked glucose branches extending from this backbone. While this is the basic structure for the presently described class of β-glucans, some variations may exist. For example, certain yeast β-glucans have additional regions of β(1,3) branching extending from the β(1,6) branches, which add further complexity to their respective structures.

β-glucans derived from baker's yeast, *Saccharomyces cerevisiae,* are made up of chains of D-glucose molecules connected at the 1 and 3 positions, having side chains of glucose attached at the 1 and 6 positions. Yeast-derived β-glucan is an insoluble, fiber-like, complex sugar having the general structure of a linear chain of glucose units with a β-1,3 backbone interspersed with β-1,6 side chains that are generally 6-8 glucose units in length. More specifically, β-glucan derived from baker's yeast is poly-(1,6)-β-D-glucopyranosyl-(1,3)-β-D-glucopyranose.

Furthermore, β-glucans are well tolerated and do not produce or cause excess gas, abdominal distension, bloating or diarrhea in pediatric subjects. Addition of β-glucan to a nutritional composition for a pediatric subject, such as an infant formula, a growing-up milk or another children's nutritional product, will improve the subject's immune response by increasing resistance against invading pathogens and therefore maintaining or improving overall health.

In an embodiment, the nutritional composition(s) of the present disclosure comprises choline. Choline is a nutrient that is essential for normal function of cells. It is a precursor for membrane phospholipids, and it accelerates the synthesis and release of acetylcholine, a neurotransmitter involved in memory storage. Moreover, though not wishing to be bound by this or any other theory, it is believed that dietary choline and docosahexaenoic acid (DHA) act synergistically to promote the biosynthesis of phosphatidylcholine and thus help promote synaptogenesis in human subjects. Additionally, choline and DHA may exhibit the synergistic effect of promoting dendritic spine formation, which is important in the maintenance of established synaptic connections. In some embodiments, the nutritional composition(s) of the present disclosure includes about 40 mg choline per serving to about 100 mg per 8 oz. serving.

In an embodiment, the nutritional composition comprises a source of iron. In an embodiment, the source of iron is ferric pyrophosphate, ferric orthophosphate, ferrous fumarate or a mixture thereof and the source of iron may be encapsulated in some embodiments.

One or more vitamins and/or minerals may also be added in to the nutritional composition in amounts sufficient to supply the daily nutritional requirements of a subject. It is to be understood by one of ordinary skill in the art that vitamin and mineral requirements will vary, for example, based on the age of the child. For instance, an infant may have different vitamin and mineral requirements than a child between the ages of one and thirteen years. Thus, the embodiments are not intended to limit the nutritional composition to a particular age group but, rather, to provide a range of acceptable vitamin and mineral components.

In embodiments providing a nutritional composition for a child, the composition may optionally include, but is not limited to, one or more of the following vitamins or derivations thereof: vitamin B₁ (thiamin, thiamin pyrophosphate, TPP, thiamin triphosphate, TTP, thiamin hydrochloride, thiamin mononitrate), vitamin B₂ (riboflavin, flavin mononucleotide, FMN, flavin adenine dinucleotide, FAD, lactoflavin, ovoflavin), vitamin B₃ (niacin, nicotinic acid, nicotinamide, niacinamide, nicotinamide adenine dinucleotide, NAD, nicotinic acid mononucleotide, NicMN, pyridine-3-carboxylic acid), vitamin B₃-precursor tryptophan, vitamin B₆ (pyridoxine, pyridoxal, pyridoxamine, pyridoxine hydrochloride), pantothenic acid (pantothenate, panthenol), folate (folic acid, folacin, pteroylglutamic acid), vitamin B₁₂ (cobalamin, methylcobalamin, deoxyadenosylcobalamin, cyanocobalamin, hydroxycobalamin, adenosylcobalamin), biotin, vitamin C (ascorbic acid), vitamin A (retinol, retinyl acetate, retinyl palmitate, retinyl esters with other long-chain fatty acids, retinal, retinoic acid, retinol esters), vitamin D (calciferol, cholecalciferol, vitamin D₃, 1,25,-dihydroxyvitamin D), vitamin E (α-tocopherol, α-tocopherol acetate, α-tocopherol succinate, α-tocopherol nicotinate, α-tocopherol), vitamin K (vitamin K₁, phylloquinone, naphthoquinone, vitamin K₂, menaquinone-7, vitamin K₃, menaquinone-4, menadione, menaquinone-8, menaquinone-8H, menaquinone-9, menaquinone-9H, menaquinone-10, menaquinone-11, menaquinone-12, menaquinone-13), choline, inositol, β-carotene and any combinations thereof.

In embodiments providing a children's nutritional product, such as a growing-up milk, the composition may optionally include, but is not limited to, one or more of the following minerals or derivations thereof: boron, calcium, calcium acetate, calcium gluconate, calcium chloride, calcium lactate, calcium phosphate, calcium sulfate, chloride, chromium, chromium chloride, chromium picolonate, copper, copper sulfate, copper gluconate, cupric sulfate, fluoride, iron, carbonyl iron, ferric iron, ferrous fumarate, ferric orthophosphate, iron trituration, polysaccharide iron, iodide, iodine, magnesium, magnesium carbonate, magnesium hydroxide, magnesium oxide, magnesium stearate, magnesium sulfate, manganese, molybdenum, phosphorus, potassium, potassium phosphate, potassium iodide, potassium chloride, potassium acetate, selenium, sulfur, sodium, docusate sodium, sodium chloride, sodium selenate, sodium molybdate, zinc, zinc oxide, zinc sulfate and mixtures thereof. Non-limiting exemplary derivatives of mineral compounds include salts, alkaline salts, esters and chelates of any mineral compound.

The minerals can be added to growing-up milks or to other children's nutritional compositions in the form of salts such as calcium phosphate, calcium glycerol phosphate, sodium citrate, potassium chloride, potassium phosphate, magnesium phosphate, ferrous sulfate, zinc sulfate, cupric sulfate, manganese sulfate, and sodium selenite. Additional vitamins and minerals can be added as known within the art.

In an embodiment, the children's nutritional composition may contain between about 10 and about 50% of the maximum dietary recommendation for any given country, or between about 10 and about 50% of the average dietary recommendation for a group of countries, per serving of vitamins A, C, and E, zinc, iron, iodine, selenium, and choline. In another embodiment, the children's nutritional composition may supply about 10 - 30% of the maximum dietary recommendation for any given country, or about 10 - 30% of the average dietary recommendation for a group of countries, per serving of B-vitamins. In yet another embodiment, the levels of vitamin D, calcium, magnesium, phosphorus, and potassium in the children's nutritional product may correspond with the average levels found in milk. In other embodiments, other nutrients in the children's nutritional composition may be present at about 20% of the maximum dietary recommendation for any given country, or about 20% of the average dietary recommendation for a group of countries, per serving.

The children's nutritional composition of the present disclosure may optionally include one or more of the following flavoring agents, including, but not limited to, flavored extracts, volatile oils, cocoa or chocolate flavorings, peanut butter flavoring, cookie crumbs, vanilla or any commercially available flavoring. Examples of useful flavorings include, but are not limited to, pure anise extract, imitation banana extract, imitation cherry extract, chocolate extract, pure lemon extract, pure orange extract, pure peppermint extract, honey, imitation pineapple extract, imitation rum extract, imitation strawberry extract, or vanilla extract; or volatile oils, such as balm oil, bay oil, bergamot oil, cedarwood oil, cherry oil, cinnamon oil, clove oil, or peppermint oil; peanut butter, chocolate flavoring, vanilla cookie crumb, butterscotch, toffee, and mixtures thereof. The amounts of flavoring agent can vary greatly depending upon the flavoring agent used. The type and amount of flavoring agent can be selected as is known in the art.

The nutritional compositions of the present disclosure may optionally include one or more emulsifiers that may be added for stability of the final product. Examples of suitable emulsifiers include, but are not limited to, lecithin (e.g., from egg or soy), alpha lactalbumin and/or mono- and di-glycerides, and mixtures thereof. Other emulsifiers are readily apparent to the skilled artisan and selection of suitable emulsifier(s) will depend, in part, upon the formulation and final product.

The nutritional compositions of the present disclosure may optionally include one or more preservatives that may also be added to extend product shelf life. Suitable preservatives include, but are not limited to, potassium sorbate, sodium sorbate, potassium benzoate, sodium benzoate, calcium disodium EDTA, and mixtures thereof.

The nutritional compositions of the present disclosure may optionally include one or more stabilizers. Suitable stabilizers for use in practicing the nutritional composition of the present disclosure include, but are not limited to, gum arabic, gum ghatti, gum karaya, gum tragacanth, agar, furcellaran, guar gum, gellan gum, locust bean gum, pectin, low methoxyl pectin, gelatin, microcrystalline cellulose, CMC (sodium carboxymethylcellulose), methylcellulose hydroxypropyl methyl cellulose, hydroxypropyl cellulose, DATEM (diacetyl tartaric acid esters of mono- and diglycerides), dextran, carrageenans, and mixtures thereof.

The nutritional compositions of the disclosure may provide minimal, partial or total nutritional support. The compositions may be nutritional supplements or meal replacements. The compositions may, but need not, be nutritionally complete. In an embodiment, the nutritional composition of the disclosure is nutritionally complete and contains suitable types and amounts of lipid, carbohydrate, protein, vitamins and minerals. The amount of lipid or fat typically can vary from about 2 to about 7 g/100 kcal. The amount of protein typically can vary from about 1 to about 5 g/100 kcal. The amount of carbohydrate typically can vary from about 8 to about 14 g/100 kcal.

In some embodiments, the nutritional composition of the present disclosure is a growing-up milk. Growing-up milks are fortified milk-based beverages intended for children over 1 year of age (typically from 1-6 years of age). They are not medical foods and are not intended as a meal replacement or a supplement to address a particular nutritional deficiency. Instead, growing-up milks are designed with the intent to serve as a complement to a diverse diet to provide additional insurance that a child achieves continual, daily intake of all essential vitamins and minerals, macronutrients plus additional functional dietary components, such as non-essential nutrients that have purported health-promoting properties.

The aforementioned nutritional compositions are capable of promoting phase II enzyme gene expression in infants or children. In some embodiments, the compositions further promote and/or modulate phase II enzyme protein expression in infants or children. In some embodiments, the nutritional composition is designed to work in concert with a regular, daily diet in order to support metabolic programming in pediatric subjects. The metabolic programming effect may evident throughout childhood as well as adulthood, thereby providing increased protection against harmful xenobiotics throughout the subject's lifetime. The aforementioned phase II enzyme may be, without limitation, glutathione transferases, UDP-glucuronosyltransferases, NAD(P)H:quinine oxireductase 1, sulfotransferases, N- & O- methyl transferases or mixtures thereof.

The exact composition of an infant formula or a growing-up milk or other nutritional composition according to the present disclosure can vary from market-to-market, depending on local regulations and dietary intake information of the population of interest. In some embodiments, nutritional compositions according to the disclosure consist of a milk protein source, such as whole or skim milk, plus added sugar and sweeteners to achieve desired sensory properties, and added vitamins and minerals. The fat composition is typically derived from the milk raw materials. Total protein can be targeted to match that of human milk, cow milk or a lower value. Total carbohydrate is usually targeted to provide as little added sugar, such as sucrose or fructose, as possible to achieve an acceptable taste. Typically, Vitamin A, calcium and Vitamin D are added at levels to match the nutrient contribution of regional cow milk. Otherwise, in some embodiments, vitamins and minerals can be added at levels that provide approximately 20% of the dietary reference intake (DRI) or 20% of the Daily Value (DV) per serving. Moreover, nutrient values can vary between markets depending on the identified nutritional needs of the intended population, raw material contributions and regional regulations.

In an embodiment, the present disclosure relates to a supplement for a pregnant or lactating female comprising a nutritional composition having a composition as defined in any one of claims 1-11, wherein the supplement promotes phase II enzyme gene expression in a prenatal infant of the pregnant female or in an infant nursing from the pregnant female. The phytonutrient may comprise any of the aforementioned phytonutrients and mixtures thereof. When administered to the lactating or pregnant female, the supplement is capable of promoting phase II enzyme gene expression in an infant nursing from the lactating female, or in a prenatal infant of the pregnant female. Exemplary phase II enzymes include, without limitation, GST, UGT, NQO1, sulfotransferases, N- & O- methyl transferases and mixtures thereof. The supplement further may be administered to a female who may become pregnant.

In an embodiment, the supplement for pregnant and lactating females further comprises any additional nutrients, including vitamins, minerals and fatty acids, that are useful in promoting the health of the pregnant or lactating female and her infant. In an embodiment of the present invention, the prenatal dietary supplement contains between about 0.1 and 10 mg folic acid. In another embodiment of the invention, the prenatal dietary supplement contains between about 0.3 and 5 mg folic acid. In a particular embodiment, the prenatal dietary supplement contains between about 0.4 and 1 mg folic acid. In yet another embodiment, the prenatal dietary supplement contains between about 400 and 700 µg per day. In a particular embodiment, the prenatal dietary supplement contains about 600 µg per day.

The prenatal dietary supplement of the invention may be administered in one or more doses daily. In some embodiments, the prenatal dietary supplement is administered in two doses daily. In a separate embodiment, the prenatal dietary supplement is administered in three daily doses.

Any orally acceptable dosage form is contemplated by the invention. Examples of such dosage forms include, but are not limited to pills, tablets, capsules, liquids, liquid concentrates, powders, elixirs, solutions, suspensions, emulsions, lozenges, beads, cachets, and combinations thereof. Alternatively, the prenatal dietary supplement of the invention may be added to a more complete nutritional product. In this embodiment, the nutritional product may contain protein, fat, and carbohydrate components and may be used to supplement the diet or may be used as the sole source of nutrition.

The present disclosure also provides non-therapeutic methods for metabolic programming of phase II enzymes. In an embodiment, the disclosure relates to a non-therapeutic method of promoting phase II enzyme gene expression in a pediatric subject, comprising administering to the subject a nutritional composition having a composition as defined in any one of claims 1-11. In an embodiment, the method further promotes phase II enzyme protein expression in the pediatric subject. The phytonutrient administered to the subject may include any of the aforementioned phytonutrients and combinations thereof. In an embodiment, the method provides increased protection from xenobiotics during childhood, when the pediatric subject becomes an adult, or both. The phase II enzyme may be, without limitation, selected from the group consisting of glutathione transferase, UDP-glucuronosyltransferase, NAD(P)H:quinine oxireductase 1, sulfotransferases, N- & O- methyl transferases and a mixture thereof.

The pediatric subject may be a child or an infant. For example, the subject may an infant ranging in age from 0 to 3 months, about 0 to 6 months, 0 to 12 months, 3 to 6 months, or 6 to 12 months. The subject may alternatively be a child ranging in age from 1 to 13 years, 1 to 6 years or 1 to 3 years. In an embodiment, the composition may be administered to the pediatric subject prenatally, during infancy, and during childhood.

The present disclosure also provides a non-therapeutic method of promoting phase II enzyme gene expression in an infant nursing from a lactating female, comprising administering to the lactating female an effective amount of a nutritional composition having a composition as defined in any one of claims 1-11 and feeding the infant milk from the lactating female. In an embodiment, the method further promotes phase II enzyme protein expression. The infant may nurse directly from the lactating female, or the milk of the lactating female may be expressed and then administered to the infant.

The present disclosure also provides a non-therapeutic method of promoting phase II enzyme gene expression in a prenatal infant, comprising administering to a female pregnant with the infant an effective amount of a nutritional composition having a composition as defined in any one of claims 1-11. In another embodiment, the disclosure provides a non-therapeutic method of promoting phase II enzyme protein expression in a prenatal infant, comprising administering to a female pregnant with the infant an effective amount of a nutritional composition having a composition as defined in any one of claims 1-11.

The phase II enzymes in the metabolic programming methods include, without limitation, glutathione transferases, UDP-glucuronosyltransferases, NAD(P)H:quinine reductase, sulfotransferases, N- & O- methyl transferases and mixtures thereof.

While not being bound by any particular theory, it is believed that the present methods provide metabolic programming effects in infants and children that will advantageously improve the subjects' ability metabolize xenobiotics throughout life. For example, by promoting phase II enzyme gene and or protein expression early in life, such as prenatally, during infancy and in childhood, the subject may have increased protection from potentially harmful xenobiotics later throughout childhood and adulthood. The improved ability to metabolize and remove xenobiotics will thus provide increased protection against diseases and conditions that are modulated by harmful xenobiotics.

The phytonutrient is selected from the group consisting of a polyphenol, a carotenoid, an isothiocyanate, or a mixture thereof. The polyphenol may be, without limitation, selected from the group consisting of flavonols, flavanols, flavanones, chalcones, flavonoids, isoflavonoids, anthocyanins, proanthocyanins, cyanidins, and mixtures thereof. The carotenoid may be, without limitation, selected from the group consisting of lutein, zeaxanthin, astaxanthin, lycopene, beta-carotene, alpha-carotene, gamma-carotene, alpha-cryptoxanthin, beta-cryptoxanthin, and mixtures thereof. The isothiocyanate may be, without limitation, selected from the group consisting of sulforaphane, phenethylisothiocyanate, and mixtures thereof. In certain embodiments, the nutritional composition comprises between about 0.01 and about 70 mg isothiocyanate per liter. In some embodiments, the phytonutrient source does not comprise soy. In still other embodiments, the phytonutrient is not a soy isoflavanoid.

The nutritional composition comprises about 50 to about 1300 nmol/L of the phytonutrient. In some embodiments, the nutritional composition comprises between about 0.01 and about 700 mg/L of the phytonutrient. Moreover, the nutritional composition may comprise about 50 to about 400 nmol/L of quercetin. In another embodiment, the composition provides about 5 to 50 mg/d of sulforaphane. In certain embodiments, the composition comprises between 0.01 and 70 mg/L of at least one isothiocyanate, such as sulforaphane. In some embodiments, the nutritional composition is formulated to deliver between about 0.01 and about 300 mg/d of the phytonutrient component. In some embodiments, the nutritional composition is formulated to deliver between about 0.01 and about 170 mg/d flavan-3-ols, such as catechins, and in certain embodiments the nutritional composition is formulated to deliver between about 0.01 and about 150 mg/d flavonols, such as quercetin.

Examples are provided to illustrate some embodiments of the nutritional composition of the present disclosure but should not be interpreted as any limitation thereon. Other embodiments within the scope of the claims herein will be apparent to one skilled in the art from the consideration of the specification or practice of the nutritional composition or methods disclosed herein. It is intended that the specification, together with the example, be considered to be exemplary only, with the scope of the disclosure being indicated by the claims which follow the example.

### EXAMPLES

### EFFECTS OF PHTYOCHEMICALS ON PHASE II ENZYME EXPRESSION IN HUMAN PRIMARY SKIN FIBROBLAST CELLS

**Materials:** Sulforaphane (4-methylsulfinylbutyl isothiocyanate; purity, 98%) is purchased from LKT laboratories (Alexis Biochemicals, UK) while catechin and quercetin are purchased from Sigma (UK). For cell cytotoxicity assays WST-1 reagent is obtained from Roche, (UK) while for quantitative PCR Bioscript RT kit, random hexamers, RNase out inhibitor and master mix reagent kit are purchase from Bioline, Promega, Invitrogen and primer design respectively. Rabbit polyclonal GSTA1 is obtained from Calbiochem and goat polyclonal NQO1 and UGT1A are obtained from Santa Cruz. All other materials and reagent unless otherwise specified are purchased from Sigma Aldrich, UK.

**Cell culture:** 1 month old (CCD-32sk), 2 year old (CCD-1092sk) and Adult (142Br) normal primary human skin fibroblast cells are obtained from the ATCC and ECACC. All cells are cultured in MEM with GluteMAX-1(GIBCO) media supplemented with 10% FBS (V/V), 1% antibiotics and 1% NEAA (GIBCO) kept at 37°C in a humidified atmosphere with 5% CO₂. Cells are media changed every 48 hours or subcultured as appropriate and used within 10 passages.

**Cytotoxicity assay:** Cell cytotoxicity following phytochemical treatment was evaluated by the WST-1 assay which measures the activity of mitochondrial dehydrogenases. Tetrazolium salts are cleaved by the dehydrogenases of viable cells to produce formazan and the change of absorbance is detected. Briefly, cells are seeded at 2×10⁴/well onto a 96 well plate and allowed to adhere overnight. Cells are then treated for 24hrs with 5, 10, 25, 50 or 100µM sulforaphane, catechin or quercetin plus no treatment control. At the end of the treatment periods, 10µl of WST-1 reagent is added to each well, and the plate is incubated for 2hr at 37°C in a humidified atmosphere of 95% air, 5% CO₂. The absorbance is measured at 450nm and the average of three blank wells containing medium and WST-1 reagent alone is subtracted from each absorbance reading. The resulting values are used for data analysis.

**RNA extraction and analysis by TaqMan real-time PCR:** Total cellular RNA is isolated using Genelute Mammalian Total RNA Kit (Sigma-Aldrich) according to the manufacturer's instructions. Total RNA is quantified (260/280nm ratio) using NanoDrop spectrophotometer (Labtech International, UK) and up to 1µg RNA is reverse transcribed using Bioscript RT kit plus random hexamers and RNase OUT inhibitor Expression of mRNA is determined by TaqMan real-time PCR using the ABI prism 7500 Sequence Detection System (Applied Biosystems). PCR reactions are carried out in a 96-well plate using master mix reagent kit in a total volume of 25µL/well consisting of 1 or 5ng of sample as appropriate, 100nmol/L probe labeled with 5' reporter dye FAM (6-carboxyfluoroscein) and 3' quencher TAMRA (6-carboxytetramethylrhodamine) and 200nmol/L forward and reverse primers. Standard curves are constructed with serial dilutions of control sample and analyzed using ABI software. Data are normalized against a house keeping gene, 18S ribosomal RNA. Gene expression is quantified by ^{ΔΔ}Ct method where fold of induction = 2^{-ΔΔCt(control)-Ct(treatment)} (Livak, K.J., Schmittgen, T.D., Analysis of relative gene expression data using real-time quantitative PCR and the 2(-Delta Delta C(T)) Method. Methods 2001. 25, 402-408).

**Preparation of protein extracts and immunoblotting:** Treated and control cells are washed twice with ice cold phosphate buffered saline (PBS) and then incubated for 30min in the Nonidet P-40(NP-40) buffer (20mM Tris-HCl, pH 8, 150mM NaCl, 10% glycerol, 1%NP-40) containing one tablet of complete mini-EDTA-free protease inhibitor cocktail (Roche) in 10 ml buffer. Cells are harvested by scraping and the homogenate is centrifuged at 13,684g at 4°C for 15 min. The supernatants are collected and frozen at -80°C. The protein concentrations are determined using Bradford reagent (Sigma) according to the manufacturer's instructions. 20µg of protein lysate is resolved by 10% SDS-polyacrylamide gel and transferred onto polyvinylidenedifluoride membranes (Bio-Rad) with a semidry transfer cell (Trans-Blot; Bio-Rad). Membranes are blocked for 1hour at room temperature or overnight at 4°C with Marvel fat-free milk powder (5% w/v), Tween 20 (0.05%, v/v) in PBS. Proteins of interest are visualized by exposing membranes to primary antibodies in milk for 2 hours at room temperature. Dilutions of antibodies are rabbit polyclonal GSTA1, 1:2000, goat polyclonal NQO1 1:1000 and goat polyclonal UGT1A 1:1000. Following primary antibody incubation membranes are incubated with suitable HRP-conjugated secondary antibody and signals are detected using an enhanced chemiluminescence kit (GE Healthcare) according to manufacturer's instructions. β-actin level is determined as loading control and bands are visualized using Fujifilm LAS3000 Imager.

**Statistical Analysis:** Statistical analysis is performed using the statistical software SPSS (version 13.1). To assess effects of various treatments, a 1-way ANOVA followed by post hoc test T-test is used. Differences are considered significant if *P* < 0.05. Results are expressed as mean with SEM of three separate experiments unless otherwise stated.

### RESULTS

**Cytotoxicity of quercetin, catechin and sulforaphane:** *In vitro* cell models of early infancy (1 month and 2 year old) and a comparative adult model are used to assess the effects of quercetin, catechin and sulforaphane on phase II enzyme expression. A range of phytochemical concentrations (1-100µM) are applied to each cell model to examine dose-response and determine optimal concentrations for future experiments. WST-1 cell viability experiments demonstrated that cells isolated from 1 month old (Fig. 1A) and adult (Fig. 1C) donors tolerate up to 50µM concentrations of all candidate phytochemicals with at least 80% viability. Cells isolated from the 2 year old donor (Fig. 1B) tolerate up to 50µM concentrations of quercetin and catechin with at least 80% viability. But, incubation with sulforaphane induces significant cell death at the 50 and 100µM concentration range. On the basis of these data future experiments are conducted at 5, 10 and 20µM to avoid deleterious effects of high phytochemical doses on cells.

**Quercetin differentially affects mRNA phase II enzymes in** 1 **month old cell model:** Cells from the 1 month old model incubated with quercetin (Fig. 2A black bars) demonstrate significant dose-response upregulation of GST (3.9-Fold) and NQO1 mRNA (7.2-Fold) compared to control (P<0.05). Similarly, and at the highest concentration of quercetin (20 µM) cells from the adult cell model also demonstrate upregulation of GST (7.4-fold) and UGT (5.5-fold) (Figure 2A open bars). In contrast, cells obtained from the 2 year old cell model do not demonstrate any significant changes in GST, UGT or NQO1 mRNA expression at all quercetin concentration tested (Fig. 2A grey bars).

**GST and NQO1 mRNA expression in infant cell lines is significantly upregulated in response to catechin:** Cells from the adult cell model (Fig 2B open bars) demonstrate dose-response upregulation of GST and NQO1 mRNA in response to catechin treatment with UGT also exhibiting an increase. The 1 month old cell model exhibits significant increases in GST mRNA expression (3.7-fold) but the NQO1 and UGT mRNA expression are unaffected. The 2 year old cell model exhibits a significant increase in NQO1 expression (4.5-fold) following catechin treatment.

**Infant cell lines exhibit significantly increased expression of NQO1 mRNA following sulforaphane treatment:** Cells from the adult cell model (Fig. 2C open bars) demonstrate a significant increase in GST, UGT and NQO1 mRNA levels; in the infant cell models in particular, significant increases in NQO1 (6-35 fold increases) are observed following sulforaphane treatment (Figure 2C black and grey bars) compared to control. GST mRNA expression is also significantly increased in response to sulforaphane in infant cell models but not to the same extent as NQO1.

**Protein expression in infant cell models:** In the one month old cell model, the expression of NQO1, UGT and GST do not demonstrate significant responses to the three phytochemicals apart from sulforaphane, which demonstrates significant induction of UGT expression (Fig. 3A). In the 2 year old cell model, protein expression of all enzymes is induced by phytochemicals except for quercetin at 10 µM. In addition, expression of UGT, GST and NQO1 proteins are significantly upregulated in response to higher doses of catechin and sulforaphane (Fig.3B). In the adult cell model, significant upregulation of NQO1 protein expression is observed in response to sulforaphane and higher dose of quercetin and catechin treatments (Fig. 3C). Expression of UGT is affected by sulforaphane treatment while GST expression increases in response to both quercetin and catechin (Fig. 3C).

The discussion of the references herein is intended merely to summarize the assertions made by their authors and no admission is made that any reference constitutes prior art. Applicants reserve the right to challenge the accuracy and pertinence of the cited references.

## Claims

1. A milk-based nutritional composition for use in promoting phase II enzyme gene expression in a pediatric subject, the composition comprising:
a fat source,
a carbohydrate source,
a protein source, and
a phytonutrient source comprising a polyphenol, an isothiocyanate, a carotenoid or a mixture thereof, wherein the composition comprises 50 to 1300 nmol/L of the phytonutrient source.

2. The milk-based nutritional composition for use of claim 1, wherein the composition further promotes phase II enzyme protein expression.

3. The milk-based nutritional composition for use of claim 2, wherein the phase II enzyme is selected from the group consisting of glutathione transferase, UDP-glucuronosyltransferase, NAD(P)H:quinone oxidoreductase 1, sulfotransferases, N-& O-methyl transferases and a mixture thereof.

4. The milk-based nutritional composition for use of claim 1, wherein the polyphenol is selected from the group consisting of flavonols, flavanols, flavan-3-ols, flavones, flavanones, chalcones, flavonoids, isoflavonoids, anthocyanins, proanthocyanidins, cyanidins, and mixtures thereof.

5. The milk-based nutritional composition for use of claim 1, wherein the carotenoid is selected from the group consisting of lutein, zeaxanthin, astaxanthin, lycopene, beta-carotene, alpha-carotene, gamma-carotene, alpha cryptoxanthin, beta cryptoxanthin and mixtures thereof.

6. The milk-based nutritional composition for use of claim 1, wherein the isothiocyanate is selected from the group consisting of sulforaphane, phenethylisothiocyanate, and mixtures thereof.

7. The milk-based nutritional composition for use of claim 1, wherein the composition comprises from 500 to 2000 nmol/L of catechins.

8. The milk-based nutritional composition for use of claim 1, wherein the composition comprises 50 to 400 nmol/L of quercetin.

9. The milk-based nutritional composition for use of claim 1, wherein the composition comprises from 0.01 to 70 mg/L of sulforaphane.

10. The milk-based nutritional composition for use of claim 1, further comprising a prebiotic.

11. The milk-based nutritional composition for use of claim 1, further comprising β-glucan.

12. A non-therapeutic method of promoting phase II enzyme gene expression in a pediatric subject, comprising administering to the subject a nutritional composition having a composition as defined in any one of claims 1-11.

13. A non-therapeutic method of promoting phase II enzyme gene expression in an infant nursing from a lactating female, comprising administering to the lactating female a nutritional composition having a composition as defined in any one of claims 1-11, and feeding the infant with breast milk from the lactating female.

14. A non-therapeutic method of promoting phase II enzyme gene expression in a prenatal infant, comprising administering to a female pregnant with the prenatal infant an effective amount of a nutritional composition having a composition as defined in any one of claims 1-11.

15. A supplement for a pregnant or lactating female, the supplement comprising a nutritional composition having a composition as defined in any one of claims 1-11, wherein the supplement is capable to promoting phase II enzyme gene expression in: (a) a prenatal infant of the pregnant female, or (b) an infant nursing from the lactating female.

## Patentansprüche

1. Nährstoffzusammensetzung auf Milchbasis zur Verwendung bei der Förderung der Genexpression von Phase II-Enzym in einem pädiatrischen Subjekt, die Zusammensetzung umfassend:
eine Fettquelle,
eine Kohlenhydratquelle,
eine Proteinquelle und
eine Phytonährstoffquelle, umfassend ein Polyphenol, ein Isothiocyanat, ein Carotenoid oder ein Gemisch davon, wobei die Zusammensetzung 50 bis 1300 nmol/L der Phytonährstoffquelle umfasst.

2. Nährstoffzusammensetzung auf Milchbasis zur Verwendung nach Anspruch 1, wobei die Zusammensetzung ferner Proteinexpression von Phase II-Enzym fördert.

3. Nährstoffzusammensetzung auf Milchbasis zur Verwendung nach Anspruch 2, wobei das Phase 11-Enzym ausgewählt ist aus der Gruppe, bestehend aus Glutathiontransferase, UDP-Glucuronosyltransferase, NAD(P)H:Chinonoxidoreduktase 1, Sulfotransferasen, N- & O-Methyltransferasen und einem Gemisch davon.

4. Nährstoffzusammensetzung auf Milchbasis zur Verwendung nach Anspruch 1, wobei das Polyphenol ausgewählt ist aus der Gruppe, bestehend aus Flavonolen, Flavanolen, Flavan-3-olen, Flavonen, Flavanonen, Chalkonen, Flavonoiden, Isoflavonoiden, Anthocyaninen, Proanthocyanidinen, Cyanidinen und Gemischen davon.

5. Nährstoffzusammensetzung auf Milchbasis zur Verwendung nach Anspruch 1, wobei das Carotenoid ausgewählt ist aus der Gruppe, bestehend aus Lutein, Zeaxanthin, Astaxanthin, Lycopin, Beta-Carotin, Alpha-Carotin, Gamma-Carotin, Alpha-Cryptoxanthin, Beta-Cryptoxanthin und Gemischen davon.

6. Nährstoffzusammensetzung auf Milchbasis zur Verwendung nach Anspruch 1, wobei das Isothiocyanat ausgewählt ist aus der Gruppe, bestehend aus Sulforaphan, Phenethylisothiocyanat und Gemischen davon.

7. Nährstoffzusammensetzung auf Milchbasis zur Verwendung nach Anspruch 1, wobei die Zusammensetzung von 500 bis 2000 nmol/L Catechine umfasst.

8. Nährstoffzusammensetzung auf Milchbasis zur Verwendung nach Anspruch 1, wobei die Zusammensetzung 50 bis 400 nmol/L Quercetin umfasst.

9. Nährstoffzusammensetzung auf Milchbasis zur Verwendung nach Anspruch 1, wobei die Zusammensetzung von 0,01 bis 70 mg/L Sulforaphan umfasst.

10. Nährstoffzusammensetzung auf Milchbasis zur Verwendung nach Anspruch 1, ferner umfassend ein Präbiotikum.

11. Nährstoffzusammensetzung auf Milchbasis zur Verwendung nach Anspruch 1, ferner umfassend β-Glucan.

12. Nichttherapeutisches Verfahren zur Förderung der Genexpression von Phase II-Enzym in einem pädiatrischen Subjekt, umfassend Verabreichen einer Nährstoffzusammensetzung mit einer Zusammensetzung wie in einem der Ansprüche 1-11 definiert an das Subjekt.

13. Nichttherapeutisches Verfahren zur Förderung der Genexpression von Phase II-Enzym in einem Säugling, gestillt von einer laktierenden Frau, umfassend Verabreichen einer Nährstoffzusammensetzung mit einer Zusammensetzung wie in einem der Ansprüche 1-11 definiert an die laktierende Frau und Ernähren des Säuglings mit Brustmilch von der laktierenden Frau.

14. Nichttherapeutisches Verfahren zur Förderung der Genexpression von Phase II-Enzym in einem pränatalen Säugling, umfassend Verabreichen einer wirksamen Menge einer Nährstoffzusammensetzung mit einer Zusammensetzung wie in einem der Ansprüche 1-11 definiert an eine Frau, die mit dem pränatalen Säugling schwanger ist.

15. Nahrungsergänzungsmittel für eine schwangere oder laktierende Frau, das Nahrungsergänzungsmittel umfassend eine Nährstoffzusammensetzung mit einer Zusammensetzung wie in einem der Ansprüche 1-11 definiert, wobei das Nahrungsergänzungsmittel in der Lage ist, Genexpression von Phase II-Enzym zu fördern in: (a) einem pränatalen Säugling der schwangeren Frau oder (b) einem Säugling, gestillt von der laktierenden Frau.

## Revendications

1. Une composition nutritionnelle à base de lait pour une utilisation destinée à promouvoir l'expression du gène de l'enzyme de phase II chez un sujet relevant de pédiatrie, la composition comprenant :
- une source de matières grasses,
- une source de glucides,
- une source de protéines, et
- une source de phytonutriments, comprenant un polyphénol, un isothiocyanate, un caroténoïde ou un mélange de ceux-ci
ladite composition comprenant 50 à 1300 nmol/ℓ de la source de phytonutriments.

2. La composition nutritionnelle à base de lait pour une utilisation selon la revendication 1, laquelle composition favorise en outre l'expression protéique de l'enzyme de phase II.

3. La composition nutritionnelle à base de lait pour une utilisation selon la revendication 2, dans laquelle l'enzyme de phase II est choisie dans le groupe consistant en glutathion transférase, UDP-glucuronosyltransférase, NAD(P)H:quinone oxydoréductase 1, sulfo-transférases, N- et O- méthyl-transférases et des mélanges de ceux-ci.

4. La composition nutritionnelle à base de lait pour une utilisation selon la revendication 1, dans laquelle le polyphénol est choisi dans le groupe constitué par les flavonols, flavanols, flavan-3-ols, les flavones, les flavanones, les chalcones, les flavonoïdes, les isoflavonoïdes, les anthocyanines, les proanthocyanidines, cyanidines, et des mélanges de ceux-ci.

5. La composition nutritionnelle à base de lait pour une utilisation selon la revendication 1, dans laquelle le caroténoïde est choisi dans le groupe constitué par la lutéine, la zéaxanthine, l'astaxanthine, le lycopène, le bêta-carotène, l'alpha-carotène, le gamma-carotène, l'alpha cryptoxanthine, la bêta cryptoxanthine et des mélanges de ceux-ci.

6. La composition nutritionnelle à base de lait pour une utilisation selon la revendication 1, dans laquelle l'isothiocyanate est choisi dans le groupe constitué par le sulforaphane, le phénéthylisothiocyanate et des mélanges de ceux-ci.

7. La composition nutritionnelle à base de lait pour une utilisation selon la revendication 1, laquelle composition comprend de 500 à 2000 nmoles/ℓ de catéchines.

8. La composition nutritionnelle à base de lait pour une utilisation selon la revendication 1, laquelle composition comprend de 50 à 400 nmol/ℓ de quercétine

9. La composition nutritionnelle à base de lait pour une utilisation selon la revendication 1, laquelle composition comprend de 0,01 à 70 mg/ℓ de sulforaphane.

10. La composition nutritionnelle à base de lait pour une utilisation selon la revendication 1, comprenant en outre un prébiotique.

11. La composition nutritionnelle à base de lait pour une utilisation selon la revendication 1, comprenant en outre du β-glucane.

12. Un procédé non-thérapeutique pour favoriser l'expression du gène de l'enzyme de phase II chez un sujet relevant de pédiatrie, comprenant l'administration au sujet d'une composition nutritionnelle ayant une composition telle que définie selon l'une quelconque des revendications 1 à 11.

13. Un procédé non-thérapeutique pour favoriser l'expression du gène de l'enzyme de phase II chez un nourrisson nourri par une femme qui l'allaite, comprenant l'administration à la femme qui l'allaite d'une composition nutritionnelle ayant une composition telle que définie selon l'une quelconque des revendications 1 à 11 et l'alimentation du nourrisson avec le lait maternel de la femme qui l'allaite.

14. Un procédé non-thérapeutique pour favoriser l'expression du gène de l'enzyme de phase II chez un enfant préalablement à sa naissance, comprenant l'administration à une femme enceinte dudit enfant d'une quantité efficace d'une composition nutritionnelle ayant une composition telle que définie selon l'une quelconque des revendications 1 à 11.

15. Un complément pour une femme enceinte ou une femme qui allaite, ce complément comprenant une composition nutritionnelle ayant une composition telle que définie selon l'une quelconque des revendications 1 à 11, lequel supplément est capable de favoriser l'expression du gène de l'enzyme de phase II chez
a) l'enfant préalablement à sa naissance d'une femme enceinte, ou
b) un enfant nourri par une femme qui l'allaite.
